(19) Europäisches Patentamt
European Patent Office
Office européen des brevets

(11) EP 4 194 477 A1

(12) # EUROPEAN PATENT APPLICATION
published in accordance with Art. 153(4) EPC

(43) Date of publication:
**14.06.2023 Bulletin 2023/24**

(21) Application number: **21853826.2**

(22) Date of filing: **26.07.2021**

(51) International Patent Classification (IPC):
***C08G 59/68*** *(2006.01)* ***A61K 6/61*** *(2020.01)*
***A61K 6/891*** *(2020.01)*

(52) Cooperative Patent Classification (CPC):
**A61K 6/61; A61K 6/891; C08G 59/68**

(86) International application number:
**PCT/JP2021/027481**

(87) International publication number:
**WO 2022/030276 (10.02.2022 Gazette 2022/06)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **07.08.2020 JP 2020134745**

(71) Applicant: **Tokuyama Dental Corporation**
**Tokyo 110-0016 (JP)**

(72) Inventors:
- **MIYAKE Hideaki**
  **Tokyo 110-0016 (JP)**
- **AKIZUMI Hironobu**
  **Tokyo 110-0016 (JP)**
- **KIRA Ryuta**
  **Tokyo 110-0016 (JP)**

(74) Representative: **Gill Jennings & Every LLP**
**The Broadgate Tower**
**20 Primrose Street**
**London EC2A 2ES (GB)**

(54) **PHOTOCATIONIC CURABLE COMPOSITION AND CURABLE DENTAL COMPOSITION**

(57) To prevent gelling even during storage for a long time period under high temperature or room temperature, and to achieve rapid curing under a room-temperature condition at the time of photoirradiation, provided are a photocationically curable composition containing a cationically polymerizable monomer and photoacid generators, and being substantially free of a hydrogen ion scavenger, wherein the photoacid generators include: at least one kind of photoacid generator selected from a photoacid generator formed of an ion pair whose anion is a noncoordinating anion, and a photoacid generator formed of a zwitterionic compound whose anionic moiety is a noncoordinating anionic moiety; and at least one kind of photoacid generator selected from a photoacid generator formed of an ion pair whose anion is not a noncoordinating anion and a photoacid generator formed of a zwitterionic compound whose anionic moiety is not a noncoordinating anionic moiety, and a dental curable composition using the same.

EP 4 194 477 A1

## Description

### Technical Field

The present invention relates to a photocationically curable composition and a dental curable composition.

### Background Art

In recent years, a photocuring-type resin composition has been frequently used for, for example, bonding of electrical/electronic parts, or a coating material. The photocuring-type resin composition has many advantages as compared to a thermal curing-type adhesive. For example, the photocuring-type resin composition cures rapidly, does not require heating at the time of curing, and hence does not inflict thermal damage to an adherend, either. In addition, the photocuring-type resin composition does not harden unless irradiated with light, and hence allows a work time before curing to be arbitrarily set. Further, the photocuring-type resin composition is free of volatilization of a solvent or the like at the time of curing.
The photocuring-type resin compositions are broadly classified into a radical polymerization type and a cationic polymerization type, and a radical polymerization type using a (meth)acrylic polymerizable compound, which has good curability, has been mainly developed and used.
The photocurable resin composition has also been utilized in dental applications (for example, Patent Literature 1). Examples thereof include: a photocurable filling and restorative material called a composite resin, which is used for restoration of a tooth damaged by caries, breakage, or the like; a dental plate liner; and hybrid ceramics for crown restoration. Besides, the photocurable resin composition is widely used in dental applications because of its ease of handling. Such photocurable dental material generally contains a polymerizable monomer and a polymerization initiator, and the composite resin and the hybrid ceramics each further contain a filler. A (meth)acrylate-based radically polymerizable monomer has been used as the polymerizable monomer because of its good photopolymerizability.
However, the (meth)acrylate-based radically polymerizable monomer has a problem in that the monomer causes large polymerization shrinkage. That is, when a filling and restorative material such as a composite resin is polymerized and cured after being filled into a cavity of a tooth in need of restoration, a surface of the filled filling and restorative material is irradiated with light to initiate polymerization. Then, owing to shrinkage caused by the polymerization, a stress acts toward peeling between the cavity and the filling and restorative material filled so as to be brought into close contact with an inner wall surface of the cavity, and hence a gap is liable to be formed between the tooth and the filling and restorative material. Accordingly, there has been a demand for a curable composition that undergoes as little polymerization shrinkage as possible and hardly forms a gap at the time of curing.
As a polymerizable monomer that causes little polymerization shrinkage, there is known a cationically polymerizable monomer, such as an epoxide or vinyl ether. However, the cationically polymerizable monomer cannot be polymerized with only a photoradical polymerization initiator, such as an $\alpha$-diketone compound or an acylphosphine oxide-based compound, which is generally used for dental use, and hence a photocationic polymerization initiator is required in order to enable its polymerization.
An example of such photocationic polymerization initiator is a photoacid generator-based photopolymerization initiator making use of a photoacid generator (e.g., an iodonium salt, a sulfonium salt, or a pyridinium salt). In particular, the iodonium salt-based photopolymerization initiator has high cationic polymerization initiation activity, and hence is useful in a dental application where rapid curing needs to be performed in the mouth. In actuality, there are also proposals of a technology in which the iodonium salt-based photopolymerization initiator is applied to a dental application in combination with the cationically polymerizable monomer (See, for example, Patent Literatures 2 and 3.).
In cationic polymerization, a cation such as a hydrogen ion causes polymerization to progress, and hence the reaction is decelerated by a component that scavenges the cation. Accordingly, when photocuring needs to be performed rapidly, it is considered that it is preferred to use a photoacid generator having a low cation scavenging ability. In the case of an onium salt-type acid generator, a moiety that is likely to bind to a cation is a counteranion, and hence a noncoordinating anion having a low ability to coordinate with a cation has been desired as a counteranion having a low cation scavenging ability. Examples thereof include such anions as trifluoromethanesulfonate and tetrakispentafluorophenylborate (See Non Patent Literatures 1 and 2.). When a photoacid generator using the above-mentioned anion as its counteranion is used, a generated hydrogen ion is hardly scavenged, and hence the cationically polymerizable monomer can be rapidly cured under a room-temperature condition.
However, such photocationically curable composition has a problem in that the composition gels relatively early during storage. In view of this, various additives for suppressing the gelling of the photocationically curable composition have been investigated, and for example, a phenolic antioxidant has been proposed as such additive (Patent Literature 4).
It is conceived that suppressive effects of phenols on gelling result from suppression of a decomposition process of the photoacid generator such as the iodonium salt. However, the phenols do not have a function of scavenging a

hydrogen ion temporarily generated through the decomposition of the photoacid generator. Accordingly, when the decomposition of the photoacid generator cannot be completely suppressed, the gelling cannot be sufficiently suppressed. In view of this, there are proposals of a technology involving further adding, as a hydrogen ion scavenger, a hindered amine or a salt of an organic acid having a pKa value of 7 or less, the salt having no photoacid generating ability, to the photocationically curable composition in addition to the phenolic antioxidant (see Patent Literatures 5 and 6). Those additives scavenge hydrogen ions generated during storage, and hence have achieved long-term storage of the photocationically curable composition at relatively high temperature.

Citation List

Patent Literature

[PTL 1] JP 2004-196775 A
[PTL 2] JP 10-508067 A
[PTL 3] JP 2005-523348 A
[PTL 4] JP 2002-69269 A
[PTL 5] JP 2006-249040 A
[PTL 6] JP 2007-131841 A

Non Patent Literature

[NPL 1] "Noncoordinating Anions-Fact or Fiction? A Survey of Likely Candidates" Krossing, I.; Raabe, I. Angew. Chem. Int. Ed. 2004, 43, 2066-2090.
[NPL 2] "Taming the Cationic Beast: Novel Developments in the Synthesis and Application of Weakly Coordinating Anions" Riddlestone, Ian M.; Kraft, A.; Schaefer, J.; Krossing, I. Angew. Chem. Int. Ed. 2018, 57, 13982-14024.

Summary of Invention

Technical Problem

However, there is a demand that a photocuring-type dental curable composition have higher long-term storage stability and rapid curability from the viewpoints of, for example, convenience and a reduction in burden on a patient. Besides, the characteristics that are the long-term storage stability and the rapid curability are also widely needed in applications other than dental applications.

The present invention has been made in view of the above-mentioned circumstances, and an object of the present invention is to provide a photocationically curable composition, which does not gel even when stored for a long time period under high temperature or room temperature, and which rapidly cures under a room-temperature condition at the time of photoirradiation, and a dental curable composition using the same.

Solution to Problem

The above-mentioned object is achieved by the present invention to be described below.

That is, according to one embodiment of the present invention, there is provided a photocationically curable composition, including: a cationically polymerizable monomer (a); and photoacid generators (b), the photocationically curable composition being substantially free of a hydrogen ion scavenger, wherein the photoacid generators (b) include: at least one kind of first photoacid generator (b1) selected from the group consisting of: a photoacid generator (b1-I) formed of an ion pair of an anion and a cation, the anion being a noncoordinating anion; and a photoacid generator (b1-II) formed of a zwitterionic compound having an anionic moiety and a cationic moiety in a molecule, the anionic moiety being a noncoordinating anionic moiety; and at least one kind of second photoacid generator (b2) selected from the group consisting of: a photoacid generator (b2-I) formed of an ion pair of an anion and a cation, the anion being an anion other than a noncoordinating anion; and a photoacid generator (b2-II) formed of a zwitterionic compound having an anionic moiety and a cationic moiety in a molecule, the anionic moiety being an anionic moiety other than a noncoordinating anionic moiety.

In the photocationically curable composition according to one embodiment of the present invention, it is preferred

that the noncoordinating anion in the first photoacid generator (b1) be at least one kind of anion selected from the group consisting of: (a) perchlorate; (b) trifluoromethanesulfonate; (c) $BX_4^-$; (d) $AlX_4^-$; (e) $PX_6^-$; (f) $AsX_6^-$; and (g) $SbX_6^-$, where X in each of the chemical formulae shown in (c) to (g) represents a fluoro group, an alkyl group substituted with one or more fluorine atoms, an alkoxy group substituted with one or more fluorine atoms, or an aryl group substituted with one or more fluorine atoms, and a plurality of Xs included in each of the chemical formulae may be identical to or different from each other.

In the photocationically curable composition according to another embodiment of the present invention, it is preferred that the cationically polymerizable monomer (a) include at least one kind of compound selected from the group consisting of: an epoxy compound; and an oxetane compound.

In the photocationically curable composition according to another embodiment of the present invention, it is preferred that a blending amount of the second photoacid generator (b2) with respect to 1 mol of a blending amount of the first photoacid generator (b1) included in the photoacid generators (b) be from 0.1 mol to 0.75 mol.

In the photocationically curable composition according to another embodiment of the present invention, it is preferred that the second photoacid generator (b2) be at least one kind of salt selected from the group consisting of: an iodonium salt; and a sulfonium salt.

It is preferred that the photocationically curable composition according to another embodiment of the present invention further include at least one kind of component selected from the group consisting of: a photosensitizer; an electron-donating compound; a phenolic antioxidant; and a filler.

According to one embodiment of the present invention, there is provided a dental curable composition, including: a cationically polymerizable monomer (a); and photoacid generators (b), the dental curable composition being substantially free of a hydrogen ion scavenger, wherein the photoacid generators (b) include: at least one kind of first photoacid generator (b1) selected from the group consisting of: a photoacid generator (b1-I) formed of an ion pair of an anion and a cation, the anion being a noncoordinating anion; and a photoacid generator (b1-II) formed of a zwitterionic compound having an anionic moiety and a cationic moiety in a molecule, the anionic moiety being a noncoordinating anionic moiety; and at least one kind of second photoacid generator (b2) selected from the group consisting of: a photoacid generator (b2-I) formed of an ion pair of an anion and a cation, the anion being an anion other than a noncoordinating anion; and a photoacid generator (b2-II) formed of a zwitterionic compound having an anionic moiety and a cationic moiety in a molecule, the anionic moiety being an anionic moiety other than a noncoordinating anionic moiety.

It is preferred that the dental curable composition according to one embodiment of the present invention further include: an $\alpha$-diketone compound (c) serving as a photosensitizer; and at least one kind of filler (d) selected from the group consisting of: an inorganic filler; and an organic-inorganic composite filler.

In the dental curable composition according to another embodiment of the present invention, it is preferred that: the dental curable composition include a polyfunctional cationically polymerizable monomer having two or more functional groups in a molecule as the cationically polymerizable monomer (a); a content ratio of the polyfunctional cationically polymerizable monomer in the cationically polymerizable monomer (a) be 50 mass% or more; a content of the first photoacid generator (b1) with respect to 100 parts by mass of the cationically polymerizable monomer (a) be from 0.01 part by mass to 20 parts by mass; a content of the second photoacid generator (b2) with respect to 100 parts by mass of the cationically polymerizable monomer (a) be from 0.01 part by mass to 20 parts by mass; a content of the $\alpha$-diketone compound (c) with respect to 100 parts by mass of the cationically polymerizable monomer (a) be from 0.001 part by mass to 10 parts by mass; and a content of the filler (d) with respect to 100 parts by mass of the cationically polymerizable monomer (a) be from 50 parts by mass to 1,500 parts by mass.

## Advantageous Effects of Invention

As described above, according to the present invention, the photocationically curable composition, which does not gel even when stored for a long time period under high temperature or room temperature, and which rapidly cures under a room-temperature condition at the time of photoirradiation, and the dental curable composition using the same can be provided.

## Description of Embodiments

A photocationically curable composition according to an embodiment of the present invention is a composition containing a cationically polymerizable monomer (a) and photoacid generators (b), and being substantially free of a hydrogen ion scavenger. Herein, a great feature of the photocationically curable composition according to this embodiment lies in containing as the photoacid generators (b) two kinds of photoacid generators having characteristics different from each other (a first photoacid generator (b1) and a second photoacid generator (b2) to be described later). In view of this, general photoacid generators and a classification method therefor are described first, and then the photocationically curable composition according to this embodiment is described.

1. With regard to General Photoacid Generators

The "photoacid generator" is generally an agent formed of an ion pair of an anion and a cation or a zwitterionic compound having an anionic moiety and a cationic moiety in the molecule, the agent being capable of generating an acid through photoirradiation. A mechanism by which an acid is generated through photoirradiation is as follows: (i) when the ion pair or the zwitterionic compound decomposes by absorbing radiated light, hydrogen is abstracted from a substance (e.g., a cationically polymerizable monomer or a solvent) present around the ion pair or the zwitterionic compound; and (ii) then, a hydrogen ion (proton) produced through the hydrogen abstraction generates an acid (Bronsted acid) together with a Bronsted base derived from the anion in the ion pair or the anionic moiety in the zwitterionic compound (The anion and the anionic moiety are hereinafter sometimes collectively referred to simply as "counteranion".). Besides, in cationic polymerization, the thus generated acid or hydrogen ion causes polymerization to progress.

2. Classification Method (Classification according to Counteranion)

The cationic polymerization activity of a photoacid generator is affected by the strength and amount of the acid to be generated at the time of photoirradiation, which are significantly affected by the counteranion. For this reason, herein, from the viewpoint of the strength of the acid scavenging ability of the counteranion to be generated, the photoacid generators are classified into two kinds, i.e., the first photoacid generator (b1) and the second photoacid generator (b2), which are defined below. Herein, the "first photoacid generator (b1)" means at least one kind of photoacid generator selected from the group consisting of: a photoacid generator (b1-I) formed of an ion pair of an anion and a cation, the anion being a noncoordinating anion; and a photoacid generator (b1-II) formed of a zwitterionic compound having an anionic moiety and a cationic moiety in the molecule, the anionic moiety being a noncoordinating anionic moiety. In addition, the "second photoacid generator (b2)" means at least one kind of photoacid generator selected from the group consisting of: a photoacid generator (b2-I) formed of an ion pair of an anion and a cation, the anion being an anion other than a noncoordinating anion; and a photoacid generator (b2-II) formed of a zwitterionic compound having an anionic moiety and a cationic moiety in the molecule, the anionic moiety being an anionic moiety other than a noncoordinating anionic moiety.

The acid scavenging ability of the counteranion significantly affects the polymerization activity. With regard to the acid scavenging ability of the counteranion, it may be said that a counteranion having a stronger interaction with a cation has a higher scavenging ability, and a counteranion having a weaker interaction therewith has a lower and weaker scavenging ability. In Non Patent Literature 1 or 2, there is a description of an anion having an extremely weak interaction with a cation ("noncoordinating anion" or "weakly coordinating anion": WCA), and it may be said that the acid scavenging ability of a "noncoordinating counteranion" such as the WCA is low. In the following description, such noncoordinating counteranion is referred to as "noncoordinating anion" when serving as an anion in an ion pair, and the noncoordinating counteranion is referred to as "noncoordinating anionic moiety" when serving as an anionic moiety in a zwitterionic compound.

Examples of the "noncoordinating anion" that is considered to fall within the category of the WCA include: (a) perchlorate; (b) trifluoromethanesulfonate; (c) $BX_4^-$; (d) $AlX_4^-$; (e) $PX_6^-$; (f) $AsX_6^-$; and (g) $SbX_6^-$, where X in each of the chemical formulae shown in (c) to (g) represents a fluoro group, an alkyl group substituted with one or more fluorine atoms, an alkoxy group substituted with one or more fluorine atoms, or an aryl group substituted with one or more fluorine atoms, and a plurality of Xs included in each of the chemical formulae may be identical to or different from each other. In addition, a "noncoordinating anion" or "noncoordinating anionic moiety" having an extremely weak interaction with a cation around the same level as (a) to (g) is also encompassed in the WCA.

It is conceived that the acid scavenging ability of the counteranion affects the initiation reaction of cationic polymerization. That is, as described already, a hydrogen ion (proton) is produced at the time of photodecomposition of a photoacid generator, and generates a Bronsted acid with a Bronsted base derived from its counteranion, but when the counteranion is a noncoordinating anion or a noncoordinating anionic moiety, the generated hydrogen ion is not scavenged thereby, and hence behaves as an extremely strong acid. In addition, the acid scavenging ability of the counteranion significantly affects the growth reaction of cationic polymerization as well. That is, after cationic polymerization has been initiated by the hydrogen ion, a carbocation is generated as an intermediate in the polymerization growth reaction, and hence, when a chemical species that reduces the reactivity of the carbocation is present in the system, the cationic polymerization is suppressed. The carbocation is a kind of acid, and hence, it is conceived that, when a counteranion having a low acid scavenging ability serves as the noncoordinating anion or the noncoordinating anionic moiety, the suppression of the cationic polymerization hardly occurs. For example, the inventors of the present invention have recognized that tetrakispentafluorophenylborate is a "noncoordinating anion", and the iodonium salt thereof extremely functions as a noncoordinating anion-type photoacid generator. However, a photocationically curable composition consisting of the above-mentioned photoacid generator easily gels when stored at high temperature. In the cationically curable composition according to this embodiment, such photoacid generator formed of an ion pair whose anion is a

noncoordinating anion or a zwitterionic compound whose anionic moiety is a noncoordinating anionic moiety is used as the first photoacid generator (b1) (Such photoacid generator is hereinafter sometimes referred to as "noncoordinating anion-type photoacid generator (b1)".).

Meanwhile, when the counteranion is not a noncoordinating anion and a noncoordinating anionic moiety, the hydrogen ion to be produced at the time of photodecomposition and the carbocation to be produced at the time of the polymerization growth reaction are affected by the counteranion. For example, in the case of an iodonium salt whose counteranion is a bromide ion, the hydrogen ion and the carbocation are bonded to the bromide ion to become hydrogen bromide and an organic bromide, respectively. Those reactions reduce the reaction activity of cationic species, and hence suppress the progress of cationic polymerization. The inventors have recognized that, when an iodonium salt whose counteranion is not a noncoordinating anion is used, although gelling during storage is less liable to be caused, it is difficult to cause cationic polymerization to progress under a room-temperature condition. In the cationically curable composition according to this embodiment, such photoacid generator formed of an ion pair whose anion is not a noncoordinating anion or a zwitterionic compound whose anionic moiety is not a noncoordinating anionic moiety is used as the second photoacid generator (b2) (Such photoacid generator is hereinafter sometimes referred to as "coordinating anion-type photoacid generator (b2)".).

3. Feature of Photocationically Curable Composition according to This Embodiment

On the basis of such findings, the inventors of the present invention have found that, when the noncoordinating anion-type photoacid generator (b1) and the coordinating anion-type photoacid generator (b2) are used in combination, rapid curability and long-term storage stability can both be achieved at high levels. Thus, the inventors have completed the photocationically curable composition according to this embodiment.

That is, the greatest feature of the photocationically curable composition according to this embodiment lies in that the photoacid generators (b) include the combination of the noncoordinating anion-type photoacid generator (b1) and the coordinating anion-type photoacid generator (b2).

In the photocationically curable composition according to this embodiment, except for the above-mentioned point, those used as a cationically polymerizable monomer and photoacid generators in a related-art photocationically curable composition may be used without a particular limitation. Including those components, the photocationically curable composition according to this embodiment is described in detail below.

4. Cationically Polymerizable Monomer (a)

In the photocationically curable composition according to this embodiment, the cationically polymerizable monomer (a) serving as a polymerizable component is not particularly limited as long as the monomer is a compound to be polymerized by an acid generated through decomposition of the photoacid generators, and a known compound may be used.

Typical examples of the cationically polymerizable monomer (a) include an epoxy compound, an oxetane compound, a cyclic ether compound, a vinyl ether compound, a bicyclic orthoester compound, a cyclic acetal compound, a bicyclic acetal compound, and a cyclic carbonate compound. In particular, an epoxy compound and/or an oxetane compound is suitably used in terms of being easily available, and causing little volume shrinkage and a fast polymerization reaction.

Specific examples of the epoxy compound include: compounds each having one epoxy functional group, such as 1,2-epoxypropane, 1,2-epoxybutane, 1,2-epoxypentane, 2,3-epoxypentane, 1,2-epoxyhexane, 1,2-epoxyoctane, 1,2-epoxydecane, 1,2-epoxydodecane, 1,2-epoxytetradecane, 1,2-epoxyhexadecane, 1,2-epoxyoctadecane, butadiene monoxide, 2-methyl-2-vinyloxirane, 1,2-epoxy-5-hexene, 1,2-epoxy-7-octene, 1,2-epoxy-9-decene, epichlorohydrin, epibromohydrin, glycidol, 2-methyl glycidol, methyl glycidyl ether, ethyl glycidyl ether, glycidyl propyl ether, butyl glycidyl ether, 2-ethylhexyl glycidyl ether, cyclooctene oxide, cyclohexene oxide, cyclooctene oxide, cyclododecane epoxide, exo-2,3-epoxynorbornene, 4-vinyl-1-cyclohexene-1,2-epoxide, limonene oxide, styrene oxide, (2,3-epoxypropyl)benzene, phenyl glycidyl ether, benzyl glycidyl ether, glycidyl 2-methylphenyl ether, 4-tert-butylphenyl glycidyl ether, 4-chlorophenyl glycidyl ether, and glycidyl 4-methoxyphenyl ether; compounds each having two epoxy functional groups, such as 1,3-butadiene dioxide, 1,2,7,8-diepoxyoctane, ethylene glycol diglycidyl ether, diethylene glycol diglycidyl ether, triethylene glycol diglycidyl ether, tetraethylene glycol diglycidyl ether, neopentyl glycol diglycidyl ether, 1,3-propanediol diglycidyl ether, 1,4-butanediol diglycidyl ether, 1,5-pentanediol diglycidyl ether, 1,6-hexanediol diglycidyl ether, 1,4-cyclohexane methanol diglycidyl ether, diglycidyl glutarate, diglycidyl adipate, diglycidyl pimelate, diglycidyl suberate, diglycidyl azelate, diglycidyl sebacate, 2,2-bis[4-glycidyloxyphenyl]propane, 2,2-bis[4-glycidyloxyphenyl]hexafluoropropane, 4-vinyl-1-cyclohexene diepoxide, limonene diepoxide, 1,2,5,6-diepoxycyclooctane, 3,4-epoxycyclohexylmethyl-3,4-epoxycyclohexane carboxylate, bis(3,4-epoxycyclohexylmethyl) glutarate, bis(3,4-epoxycyclohexylmethyl) adipate, bis(3,4-epoxycyclohexylmethyl) pimelate, bis(3,4-epoxycyclohexylmethyl) suberate, bis(3,4-epoxycyclohexylmethyl)

azelate, bis(3,4-epoxycyclohexylmethyl) sebacate, 1,4-bis(3,4-epoxycyclohexylmethyloxymethyl)benzene, 1,4-bis(3,4-epoxycyclohexylmethyloxymethyl)biphenyl, bis(3,4-epoxycyclohexyl)methane, 2,2-bis(3,4-epoxycyclohexyl)propane, bis(3,4-epoxycyclohexyl)sulfone, methylbis[2-(7-oxabicyclo[4.1.0]hept-3-yl)ethyl]phenylsilane, dimethylbis[(7-oxabicyclo[4.1.0]hept-3-yl)methyl]silane, methyl[(7-oxabicyclo[4.1.0]hept-3-yl)methyl] [2-(7-oxabicyclo[4.1.0]hept-3-yl)ethyl]silane, 1,4-phenylenebis[dimethyl[2-(7-oxabicyclo[4.1.0]hept-3-yl)ethyl]]silane, 1,2-ethylenebis[dimethyl[2-(7-oxabicyclo[4.1.0]hept-3-yl)ethyl]]silane, dimethyl[2-(7-oxabicyclo[4.1.0]hept-3-yl)ethyl]silane, 1,3-bis[2-(7-oxabicyclo[4.1.0]hept-3-yl)ethyl]-1,1,3,3-tetramethyldisiloxane, 2,5-bicyclo[2.2.1]heptylenebis[dimethyl[2-(7-oxabicyclo[4.1.0]hept-3-yl)ethyl]]silane, and 1,6-hexylenebis[dimethyl[2-(7-oxabicyclo[4.1.0]hept-3-yl)ethyl]]silane; and compounds each having three or more epoxy functional groups, such as glycerol triglycidyl ether, trimethylolpropane triglycidyl ether, pentaerythritol triglycidyl ether, pentaerythritol tetraglycidyl ether, and dipentaerythritol hexaglycidyl ether.

In addition, examples of the epoxy compound also include compounds having cyclic siloxane and silsesquioxane structures and having epoxy functional groups, such as compounds shown below. In the structural formulae (except for the structural formulae for specifying R), a bonding site with *1 represents bonding to an O atom of an adjacent repeating constituent unit, and a bonding site with *2 represents bonding to a Si atom of an adjacent repeating constituent unit.

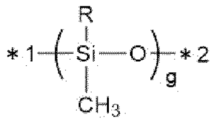

g = 3 ~ 6

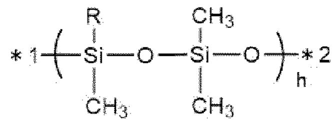

h = 2, 3

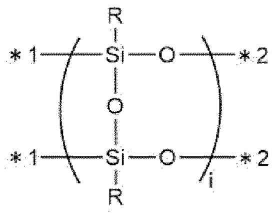

i = 3 ~ 6

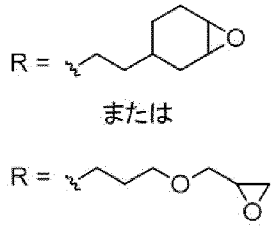

または

or

Of the above-mentioned epoxy compounds, one having two or more epoxy functional groups in one molecule is particularly suitably used in terms of the physical properties of a cured body to be obtained.

In addition, specific examples of the oxetane compound include: compounds each having one oxetane ring, such as trimethylene oxide, 3-methyl-3-oxetanylmethanol, 3-ethyl-3-oxetanylmethanol, 3-ethyl-3-phenoxymethyloxetane, 3,3-diethyloxetane, and 3-ethyl-3-(2-ethylhexyloxy)oxetane; and compounds each having two or more oxetane rings, such as 1,4-bis(3-ethyl-3-oxetanylmethyloxy)benzene, 4,4'-bis(3-ethyl-3-oxetanylmethyloxy)biphenyl, 4,4'-bis(3-ethyl-3-oxetanylmethyloxymethyl)biphenyl, ethylene glycol bis(3-ethyl-3-oxetanylmethyl) ether, diethylene glycol bis(3-ethyl-3-oxetanylmethyl) ether, bis(3-ethyl-3-oxetanylmethyl) diphenoate, trimethylolpropane tris(3-ethyl-3-oxetanylmethyl) ether, pentaerythritol tetrakis(3-ethyl-3-oxetanylmethyl) ether, and compounds shown below. In the bottom-left structural formula out of the structural formulae shown below, a bonding site with *1 represents bonding to an O atom of an adjacent repeating constituent unit, and a bonding site with *2 represents bonding to a Si atom of an adjacent repeating constituent unit.

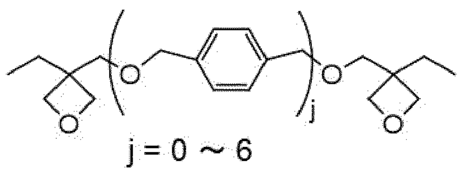

j = 0 ~ 6

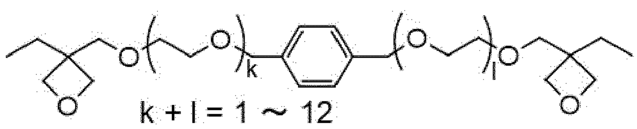

k + l = 1 ~ 12

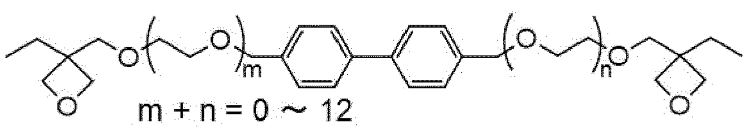

m + n = 0 ~ 12

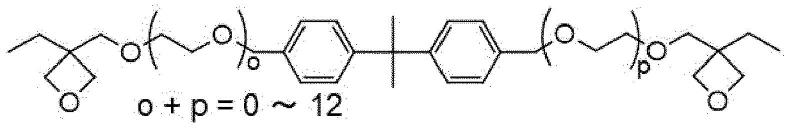

o + p = 0 ~ 12

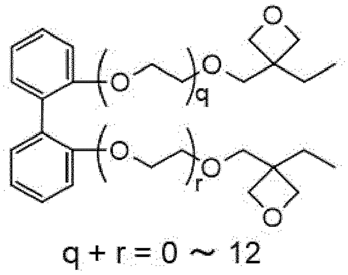

q + r = 0 ~ 12

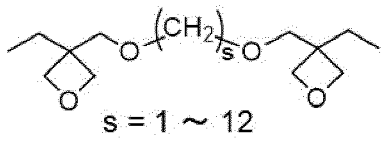

s = 1 ~ 12

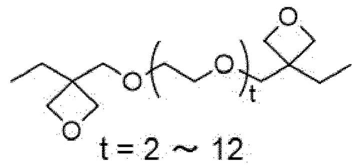

t = 2 ~ 12

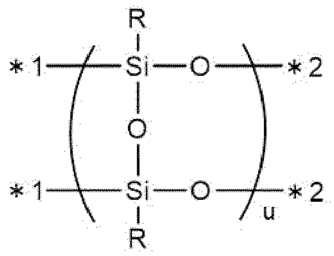

u = 3 ~ 6

R = 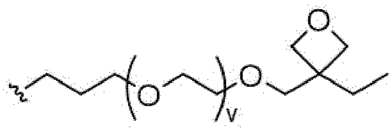

v = 0 ~ 6

Of the above-mentioned oxetane compounds, one having two or more oxetane rings (oxetane functional groups) in one molecule is particularly suitably used in terms of the physical properties of a cured body to be obtained.

In addition, specific examples of the cationically polymerizable monomer other than the oxetane compound and the epoxy compound include: cyclic ether compounds, such as tetrahydrofuran and oxepane; bicyclic orthoester compounds, such as a bicyclo orthoester, a spiro orthoester, and a spiro orthocarbonate; cyclic acetal compounds, such as 1,3,5-trioxane, 1,3-dioxolane, oxepane, 1,3-dioxepane, 4-methyl-1,3-dioxepane, and 1,3,6-trioxacyclooctane; bicyclic acetal compounds, such as 2,6-dioxabicyclo[2.2.1]heptane, 2,7-dioxabicyclo[2.2.1]heptane, and 6,8-dioxabicyclo[3.2.1]octane; cyclic carbonate compounds, such as ethylene carbonate and trimethylene carbonate; and olefinic compounds, such as vinyl ether and styrene.

Those cationically polymerizable monomers may be used alone or in combination thereof. When the photocationically curable composition according to this embodiment is used as a dental curable composition, it is preferred that: <i> the composition contain a polyfunctional cationically polymerizable monomer having two or more functional groups in the molecule as the cationically polymerizable monomer (a); and <ii> the content ratio of the polyfunctional cationically polymerizable monomer in the cationically polymerizable monomer (a) be 50 mass% or more. When those conditions <i> and <ii> are satisfied, the mechanical strength of the cured body obtained by curing the photocationically curable composition can be extremely easily made suitable for utilization with a dental purpose. The polyfunctional cationically polymerizable monomer is suitably an epoxy compound having two or more epoxy functional groups in the molecule and/or an oxetane compound having two or more oxetane functional groups in the molecule. As long as the number of functional groups that the polyfunctional cationically polymerizable monomer has in the molecule is 2 or more, the upper limit value thereof is not particularly limited, but is preferably 8 or less in practical use. In addition, from the viewpoint of suppressing the formation of a gap between the surface of a tooth to be treated and the cured body by suppressing excessive polymerization shrinkage at the time of polymerization, the upper limit value is more preferably 4 or less. In addition, the content ratio of the polyfunctional cationically polymerizable monomer in the cationically polymerizable monomer (a) is more preferably 70 mass% or more, still more preferably 90 mass% or more, and may be 100 mass%.

In addition, from the viewpoint of increasing the curing rate of the photocationically curable composition according to this embodiment to suppress polymerization failure due to moisture, it is preferred that: <A> a mixture of an oxetane compound having an oxetane functional group and an epoxy compound having an epoxy functional group be used as the cationically polymerizable monomer (a); and <B> the following equation (1) be satisfied. In this case, it is particularly suitable to further satisfy the conditions <A> and <B> in addition to satisfying the conditions <i> and <ii>.

Equation (1) $(a\times A):(b\times B)=90:10$ to $10:90$

In the equation (1), "a" represents the average number of oxetane functional groups per molecule of the oxetane compound, A represents the number of moles of the oxetane compound contained in the mixture, "b" represents the average number of epoxy functional groups per molecule of the epoxy compound, and B represents the number of moles of the epoxy compound contained in the mixture. Here, "a" and "b" each represent a number of 1 or more, preferably 1.5 or more, more preferably 2 or more. Meanwhile, the upper limit value of each of "a" and "b" is not particularly limited, but is preferably 4 or less in practical use. In addition, $(a\times A):(b\times B)$ is more preferably from 80:20 to 20:80, still more preferably from 70:30 to 30:70.

5. Photoacid Generators (b)

The photoacid generators (b) to be used in the photocationically curable composition according to this embodiment include the noncoordinating anion-type photoacid generator (b1) and the coordinating anion-type photoacid generator (b2). Consequently, as described above, rapid curability and long-term storage stability can both be achieved. When only the noncoordinating anion-type photoacid generator (b1) is used as the photoacid generator (b), the storage stability is reduced, and when only the coordinating anion-type photoacid generator (b2) is used, the curability is reduced.
The usage amount of each of the noncoordinating anion-type photoacid generator (b1) and the coordinating anion-type photoacid generator (b2) is not particularly limited as long as the amount allows polymerization to be initiated through photoirradiation, but the following conditions <iii> and <iv> are suitably satisfied in order to achieve both of an appropriate rate of progress of the polymerization and various physical properties (e.g., weather resistance and hardness) of the cured body to be obtained. That is, <iii> in terms of the content of the noncoordinating anion-type photoacid generator (b1) with respect to 100 parts by mass of the cationically polymerizable monomer (a), 0.01 part by mass to 20 parts by mass thereof is preferably used, and 0.05 part by mass to 10 parts by mass thereof is more preferably used, and <iv> in terms of the content of the coordinating anion-type photoacid generator (b2) with respect to 100 parts by mass of the cationically polymerizable monomer (a), 0.01 part by mass to 20 parts by mass thereof is preferably used, and 0.05 part by mass to 10 parts by mass thereof is more preferably used.
It should be noted that the blending amount of the coordinating anion-type photoacid generator (b2) with respect to 1 mol of the noncoordinating anion-type photoacid generator (b1) is preferably from 0.01 mol to 0.9 mol. When the blending amount of the coordinating anion-type photoacid generator (b2) falls within this range, it becomes easier to suppress gelling by interacting with cationic active species to be produced during the storage of the photocationically curable composition, and besides, cationic active species to be produced due to the noncoordinating anion-type photoacid generator (b1) are not entirely scavenged, and hence cationic polymerization progresses quickly at the time of photoirradiation to make it easier for the photocationically curable composition to rapidly cure as well. From the viewpoint that it becomes easy to achieve both storage stability and rapid curability at higher levels as just described, the blending amount of the coordinating anion-type photoacid generator (b2) with respect to 1 mol of the noncoordinating anion-type photoacid generator (b1) is more preferably from 0.05 mol to 0.8 mol, most preferably from 0.1 mol to 0.75 mol.

5-1. Noncoordinating Anion-type Photoacid Generator (b1)

In the photocationically curable composition according to this embodiment, the noncoordinating anion-type photoacid generator (b1) is not particularly limited as long as the photoacid generator is at least one kind of photoacid generator selected from the group consisting of: the photoacid generator (b1-I) formed of an ion pair of an anion and a cation, the anion being a noncoordinating anion; and the photoacid generator (b1-II) formed of a zwitterionic compound having an anionic moiety and a cationic moiety in the molecule, the anionic moiety being a noncoordinating anionic moiety, and a known compound to be used as such photoacid generator may be used.
Here, examples of the "noncoordinating anion" in the noncoordinating anion-type photoacid generator (b1) include: (a) perchlorate; (b) trifluoromethanesulfonate; (c) $BX_4^-$; (d) $AlX_4^-$; (e) $PX_6^-$; (f) $AsX_6^-$; and (g) $SbX_6^-$, where X in each of the chemical formulae shown in (c) to (g) represents a fluoro group, an alkyl group substituted with one or more fluorine atoms, an alkoxy group substituted with one or more fluorine atoms, or an aryl group substituted with one or more fluorine atoms, and a plurality of Xs included in each of the chemical formulae may be identical to or different from each other. Of those, (c) $BX_4^-$ or (d) $AlX_4^-$, where X in each of the chemical formulae shown in (c) and (d) represents an alkoxy group substituted with three or more fluorine atoms, or an aryl group substituted with three or more fluorine atoms, and a plurality of Xs included in each of the chemical formulae may be identical to or different from each other, is particularly preferred as the "noncoordinating anion".
The cation or the cationic moiety in the noncoordinating anion-type photoacid generator (b1) is required to satisfy the conditions of: having a positive charge; causing a decomposition reaction at the time of photoirradiation; and itself and its decomposition product hardly scavenging an acid. Examples of the cation that satisfies those conditions include iodonium and sulfonium, and examples of the cationic moiety that satisfies those conditions include residues each obtained by removing any one atom from a cation, such as iodonium or sulfonium. Of those, diaryliodonium and

a residue thereof, and triarylsulfonium and a residue thereof are particularly preferred because of high decomposition reaction activity and a low acid scavenging ability.

The noncoordinating anion-type photoacid generator (b1) that may be particularly suitably used may be exemplified by: a photoacid generator (b1-I) that is an onium salt compound formed of a combination of any one cation selected from a cation group described below and any one anion selected from an anion group described below; or a photoacid generator (b1-II) that is a zwitterionic compound formed of a combination of any one cationic moiety selected from a cationic moiety group described below and any one anionic moiety selected from an anionic moiety group described below.

[Cation Group]

Diphenyliodonium, bis(p-chlorophenyl)iodonium, ditolyliodonium, bis(p-tert-butylphenyl)iodonium, p-isopropylphenyl-p-methylphenyliodonium, bis(m-nitrophenyl)iodonium, p-tert-butylphenylphenyliodonium, p-methoxyphenylphenyliodonium, bis(p-methoxyphenyl)iodonium, p-octyloxyphenylphenyliodonium, p-phenoxyphenylphenyliodonium, and bis(p-dodecylphenyl)iodonium.

[Cationic Moiety Group]

Residues each obtained by removing any one atom from a cation listed in the above-mentioned cation group. In this case, "any one atom" means an atom among the constituent atoms of the cation, the atom being present at a position at which a group containing an anionic moiety can be introduced as a substituent into the cation.

[Anion Group]

Perchlorate, trifluoromethanesulfonate, tetrafluoroborate, tetrakispentafluorophenylborate, tetrakispentafluorophenylgallate, hexafluorophosphate, hexafluoroarsenate, hexafluoroantimonate, and tetrakisnonafluoro-tert-butoxyaluminate.

Of the anions shown in the above-mentioned anion group, tetrakispentafluorophenylborate or tetrakisnonafluoro-tert-butoxyaluminate is particularly suitable.

[Anionic Moiety Group]

Residues each obtained by removing any one atom from an anion listed in the above-mentioned anion group. In this case, "any one atom" means an atom among the constituent atoms of the anion, the atom being present at a position at which a group containing a cationic moiety can be introduced as a substituent into the anion.

5-2. Coordinating Anion-type Photoacid Generator (b2)

In the photocationically curable composition according to this embodiment, the coordinating anion-type photoacid generator (b2) is not particularly limited as long as the photoacid generator is at least one kind of photoacid generator selected from the group consisting of: the photoacid generator (b2-I) formed of an ion pair of an anion and a cation, the anion not being a noncoordinating anion; and the photoacid generator (b2-II) formed of a zwitterionic compound having an anionic moiety and a cationic moiety in the molecule, the anionic moiety not being a noncoordinating anionic moiety, and a known compound to be used as a photoacid generator may be used, but an iodonium salt and/or a sulfonium salt is preferably used.

The anion in the coordinating anion-type photoacid generator (b2) is not particularly limited as long as the anion is an anion other than a noncoordinating anion, and the anionic moiety in the coordinating anion-type photoacid generator (b2) is not particularly limited as long as the anionic moiety is an anionic moiety other than a noncoordinating anionic moiety. However, the anion or the anionic moiety in the coordinating anion-type photoacid generator (b2) is preferably a halide ion, a sulfonate, or a carboxylate for the reasons of being easily available and having an appropriate acid scavenging ability.

The cation or the cationic moiety in the coordinating anion-type photoacid generator (b2) is required to satisfy the conditions of: having a positive charge; causing a decomposition reaction at the time of photoirradiation; and itself and its decomposition product hardly scavenging an acid. Examples of the cation that satisfies those conditions include iodonium and sulfonium, and examples of the cationic moiety that satisfies those conditions include residues obtained by removing any one atom from iodonium and sulfonium. Of those, diaryliodonium and a residue thereof, and triarylsulfonium and a residue thereof are particularly preferred because of high decomposition reaction activity and a low acid scavenging ability.

The coordinating anion-type photoacid generator (b2) that may be particularly suitably used may be exemplified

by: a photoacid generator (b2-I) that is an onium salt compound formed of a combination of any one cation selected from a cation group described below and any one anion selected from an anion group described below; or a photoacid generator (b2-II) that is a zwitterionic compound formed of a combination of any one cationic moiety selected from a cationic moiety group described below and any one anionic moiety selected from an anionic moiety group described below.

[Cation Group]

Diphenyliodonium, bis(p-chlorophenyl)iodonium, ditolyliodonium, bis(p-tert-butylphenyl)iodonium, p-isopropylphenyl-p-methylphenyliodonium, bis(m-nitrophenyl)iodonium, p-tert-butylphenylphenyliodonium, p-methoxyphenylphenyliodonium, bis(p-methoxyphenyl)iodonium, p-octyloxyphenylphenyliodonium, p-phenoxyphenylphenyliodonium, bis(p-dodecylphenyl)iodonium, triphenylsulfonium, tritolylsulfonium, p-tert-butylphenyldiphenylsulfonium, diphenyl-4-phenylthiophenylsulfonium, diphenyl-2,4,6-trimethylphenylsulfonium, 1-methylpyridinium, and 1-methyl-2-chloropyridinium.

[Cationic Moiety Group]

Residues each obtained by removing any one atom from a cation listed in the above-mentioned cation group.

[Anion Group]

An iodide ion, a bromide ion, a chloride ion, a sulfate ion, a hydrogen sulfate ion, a carbonate ion, a hydrogen carbonate ion, methanesulfonate, benzenesulfonate, p-toluenesulfonate, nitrate, chloroacetate, acetate, formate, banzoate, and phenolate.

[Anionic Moiety Group]

Residues each obtained by removing any one atom from an anion listed in the above-mentioned anion group (except for an anion formed of only one atom, such as $Br^-$).

The above-mentioned coordinating anion-type photoacid generators (b2) may be used alone or as a mixture thereof as required without any problem.

6. Hydrogen Ion Scavenger (Component that is substantially not contained)

The photocationically curable composition according to this embodiment is substantially free of a hydrogen ion scavenger. Herein, "substantially free" means that the hydrogen ion scavenger is not contained in such an amount as to adversely affect polymerization-curability at room temperature. Specifically, "substantially free" means (i) a case in which the photocationically curable composition contains no hydrogen ion scavenger, and (ii) a case in which the photocationically curable composition contains a hydrogen ion scavenger, but its content is more than 0 ppm and 500 ppm or less with respect to the total mass of the cationically polymerizable monomer (a) {more than 0 parts by mass and 0.05 part by mass or less with respect to 100 parts by mass of the cationically polymerizable monomer (a)}. In the latter case (ii), the upper limit value of the content is preferably 100 ppm or less.

The "hydrogen ion scavenger" means a compound having a hydrogen ion scavenging ability to be used in the technology described in each of Patent Literatures 5 and 6, that is, (i) an aliphatic amine and (ii) a salt of an organic acid having a pKa value of 7 or less, the salt having no photoacid generating ability. Here, specific examples of (i) the aliphatic amine include 2,2,6,6,-tetramethylpiperidine and derivatives thereof, and N,N-diisopropylethylamine. An aromatic amine, in which a nitrogen atom and an aromatic ring are directly bonded to each other, has an extremely low hydrogen ion scavenging ability, and hence does not fall within the category of the hydrogen ion scavenger not to be used in the photocationically curable composition according to this embodiment. In addition, (ii) the salt of an organic acid having a pKa value of 7 or less, the salt having no photoacid generating ability, refers to a salt having a quantum yield of less than 0.001 for acid generation when irradiated with light having a wavelength longer than 200 nm. The quantum yield for acid generation may be measured by a method described in J Polym Sci Part A: Polym Chem 2003, 41, 2570. Specific examples of (ii) the salt of an organic acid having a pKa value of 7 or less, the salt having no photoacid generating ability, may include sodium stearate and tetraethylammonium p-toluenesulfonate. The photoacid generators (b) to be used in the photocationically curable composition according to this embodiment are also salts of organic acids, but the salts of organic acids to be used as the photoacid generators (b) fall outside the above-mentioned definition of (ii) the salt of an organic acid having a pKa value of 7 or less, the salt having no photoacid generating ability.

The hydrogen ion scavenger scavenges a hydrogen ion generated during the storage of a photocationically curable composition, and hence is advantageous for long-term storage at relatively high temperature. However, the

hydrogen ion scavenger inhibits the progress of cationic polymerization, and hence is feared to reduce the curing rate at the time of photoirradiation. For this reason, when the hydrogen ion scavenger is used, the kind and blending amount of the hydrogen ion scavenger to be used need to be selected well in order to achieve both rapid curability and long-term storage stability in a well-balanced manner. However, in light of the action and effect of the hydrogen ion scavenger described above, in a photocationically curable composition using the hydrogen ion scavenger, rapid curability and long-term storage stability are in a trade-off relationship in principle. Consequently, there is a limit in achieving both the two characteristics at higher levels. However, in the photocationically curable composition according to this embodiment, both the two characteristics can be easily achieved at higher levels by utilizing the two kinds of photoacid generators in combination while using substantially no hydrogen ion scavenger.

7. Other Components

The photocationically curable composition according to this embodiment may contain other blend components in addition to the above-mentioned components as required from, for example, purposes and applications within ranges of kinds and blending amounts that do not impair the achievement of both rapid curability and long-term storage stability. Here, when the photocationically curable composition according to this embodiment is regarded as a composition containing non-photocationic polymerization initiator components (A) including the cationically polymerizable monomer (a) and photocationic polymerization initiator components (B) including the photoacid generators (b), and being substantially free of a hydrogen ion scavenger, examples of the other components may typically include components classified below.

<Non-photocationic polymerization initiator components (A) other than cationically polymerizable monomer (a)>

·Phenolic antioxidant
·Filler
·Other additives

<Photocationic polymerization initiator components (B) other than photoacid generators (b)>

·Photosensitizer (polymerization accelerator)
·Electron-donating compound

In this case, the photocationic polymerization initiator components (B) may consist only of the photoacid generators (b) including the noncoordinating anion-type photoacid generator (b1) and the coordinating anion-type photoacid generator (b2), or may further include, in addition to the photoacid generators (b), a photosensitizer for accelerating polymerization, and an electron-donating compound, which accelerates polymerization by accelerating the decomposition of the photoacid generators. In this case, the photocationic polymerization initiator components (B) contained in the photocationically curable composition according to this embodiment are <i> consist only of the photoacid generators (b), or <ii> consist only of the photoacid generators (b) and at least one kind of compound selected from the group consisting of the photosensitizer and the electron-donating compound. Examples of the case <ii> include <iia> a combination of the photoacid generators (b) and the photosensitizer, <iib> a combination of the photoacid generators (b) and the electron-donating compound, and <iic> a combination of the photoacid generators (b), the photosensitizer, and the electron-donating compound. Of those, the combination <iia> is suitable.

7-1. Photosensitizer (Polymerization Accelerator)

As the photosensitizer to be used in the above-mentioned case <ii>, for example, a ketone compound (in particular, an $\alpha$-diketone compound) and/or a coumarin-based dye may be suitably used. In addition, when the photocationically curable composition according to this embodiment is used as a dental curable composition, an $\alpha$-diketone compound is preferably used as the photosensitizer because of its ability to accelerate the decomposition of the photoacid generators through visible photoirradiation. The $\alpha$-diketone compound fades in color by reacting with another component at the time of the curing of the photocationically curable composition, and hence is also advantageous in terms of the esthetics and color tone control of a tooth to be treated. That is, when the cationic polymerization initiators (B) contained in the photocationically curable composition according to this embodiment fall under the above-mentioned case <ii>, the photosensitizer is particularly preferably formed of the $\alpha$-diketone compound.

Specific examples of the $\alpha$-diketone compound include camphor quinone (CQ), benzil, diacetyl, acetylbenzoyl, 2,3-pentadione, 2,3-octadione, 4,4'-dimethoxybenzil, 4,4'-oxybenzil, 9,10-phenanthrenequinone, and acenaphthenequinone.

A diaryl ketone compound is specifically, for example, 4,4-bis(dimethylamino)benzophenone, 9-fluorenone,

3,4-benzo-9-fluorenone, 2-dimethylamino-9-fluorenone, 2-methoxy-9-fluorenone, 2-chloro-9-fluorenone, 2,7-dichloro-9-fluorenone, 2-bromo-9-fluorenone, 2,7-dibromo-9-fluorenone, 2-nitro-9-fluorenone, 2-acetoxy-9-fluorenone, benzanthrone, anthraquinone, 1,2-benzanthraquinone, 2-methylanthraquinone, 2-ethylanthraquinone, 1-dimethylaminoanthraquinone, 2,3-dimethylanthraquinone, 2-tert-butylanthraquinone, 1-chloroanthraquinone, 2-chloroanthraquinone, 1,5-dichloroanthraquinone, 1,2-dimethoxyanthraquinone, 1,2-diacetoxy-anthraquinone, 5,12-naphthacenequinone, 6,13-pentacenequinone, xanthone, thioxanthone, 2,4-dimethylthioxanthone, 2,4-diethylthioxanthone, 2-chlorothioxanthone, 9(10H)-acridone, 9-methyl-9(10H)-acridone, or dibenzosuberenone.

A ketocoumarin compound is, for example, 3-benzoylcoumarin, 3-(4-methoxybenzoyl)coumarin, 3-benzoyl-7-methoxycoumarin, 3-(4-methoxybenzoyl)7-methoxy-3-coumarin, 3-acetyl-7-dimethylaminocoumarin, 3-benzoyl-7-dimethylaminocoumarin, 3,3'-coumarinoketone, or 3,3'-bis(7-diethylaminocoumarino) ketone.

The above-mentioned photosensitizers may be used alone or as a mixture thereof as required without any problem. The usage amount of the photosensitizer varies depending on the kinds of other components to be combined therewith and the polymerizable monomer, but is generally preferably from 0.001 part by mass to 10 parts by mass, more preferably from 0.01 part by mass to 5 parts by mass, still more preferably from 0.05 part by mass to 2 parts by mass with respect to 100 parts by mass of the cationically polymerizable monomer (a).

7-2. Electron-donating Compound (Polymerization Accelerator)

As the electron-donating compound that functions as the polymerization accelerator, an aromatic amine, such as phenylalanine, 4-dimethylaminobenzoic acid ester, or 4-dimethylaminotoluene, or an alkoxyaryl compound, such as p-dimethoxybenzene, 1,2,4-trimethoxybenzene, or 2-ethyl-9,10-dimethoxyanthracene, is suitably used. Even when the electron-donating compound is a compound containing at least a fused polycyclic aromatic ring such as anthracene, in the photocationically curable composition according to this embodiment, a compound having a molecular structure in which a nonaromatic ring further fused to the fused polycyclic aromatic ring is fused is not used as the electron-donating compound.

The above-mentioned electron-donating compounds may be used alone or as a mixture thereof as required without any problem. The usage amount of the electron-donating compound varies depending on the kinds of other components to be combined therewith and the polymerizable monomer, but is generally preferably from 0.001 part by mass to 10 parts by mass, more preferably from 0.01 part by mass to 5 parts by mass, still more preferably from 0.05 part by mass to 2 parts by mass with respect to 100 parts by mass of the cationically polymerizable monomer (a) .

7-3. Phenolic Antioxidant

The photocationically curable composition according to this embodiment may contain a phenolic antioxidant as an additive for suppressing the decomposition of the photoacid generators. As the phenolic antioxidant, a hitherto known one may be utilized without any limitation. Examples thereof include 4-methoxyphenol, hydroquinone, 2,6-di-t-butylphenol, 2,4-di-t-butylphenol, and 2-t-butyl-4,6-dimethylphenol.

The above-mentioned phenolic antioxidants may be used alone or as a mixture thereof as required without any problem. The usage amount of the photosensitizer varies depending on the kinds of other components to be combined therewith and the polymerizable monomer, but is generally preferably from 0.001 part by mass to 10 parts by mass, more preferably from 0.01 part by mass to 5 parts by mass, still more preferably from 0.05 part by mass to 2 parts by mass with respect to 100 parts by mass of the cationically polymerizable monomer (a) .

7-4. Filler

When the photocationically curable composition according to this embodiment is used as a dental curable composition (in particular, a dental filling and restorative material), a filler is preferably blended thereinto.

As the filler, any of an organic filler, an inorganic filler, or an organic-inorganic composite filler to be blended into a known dental curable composition may be blended. Examples of the organic filler include particles formed of organic polymers, such as polymethyl methacrylate, polyethyl methacrylate, a methyl methacrylate-ethyl methacrylate copolymer, crosslinked polymethyl methacrylate, crosslinked polyethyl methacrylate, an ethylene-vinyl acetate copolymer, a styrenebutadiene copolymer, an acrylonitrile-styrene copolymer, and an acrylonitrile-butadiene-styrene copolymer.

Specific examples of the inorganic filler include inorganic particles, such as quartz, silica, alumina, silica titania, silica zirconia, lanthanum glass, barium glass, and strontium glass. In addition, examples of the organic-inorganic composite filler include particulate organic-inorganic composite particles obtained by mixing those inorganic particles with a polymerizable monomer in advance to give a paste-like mixture, followed by polymerization and pulverization. The inorganic filler to be used may contain a heavy metal such as zirconia to impart an X-ray contrast property.

The particle diameter and shape of such filler are not particularly limited, and spherical or indefinite-shaped particles having an average particle diameter of from 0.01 um to 100 um that are generally used as a dental material may be appropriately used in accordance with purposes. In addition, the refractive index of such filler is also not particularly limited, and a refractive index falling within the range of from 1.4 to 1.7, which a filler for a general dental curable composition has, may be used without any limitation.
When the above-mentioned filler is blended into the photocationically curable composition according to this embodiment, its blending amount is also not particularly limited as long as the blending amount falls within a range in which the composition becomes paste-like. However, when the photocationically curable composition according to this embodiment is used as a dental curable composition (in particular, a dental filling and restorative material), an inorganic filler and/or an organic-inorganic composite filler is preferably used as the filler. In this case, <vi> the content of the filler with respect to 100 parts by mass of the cationically polymerizable monomer (a) is preferably from 50 parts by mass to 1,500 parts by mass, more preferably from 70 parts by mass to 1,000 parts by mass.
Further, those fillers, such as inorganic fillers and organic-inorganic composite fillers, may each be used alone, or a plurality of kinds thereof different from each other in material, particle diameter, shape, or the like may be used in combination. It is particularly preferred that the inorganic filler be mainly used in the case of use as a dental filling and restorative material in terms of being excellent in mechanical physical properties after curing.
The filler affects the mechanical strength of the cured body obtained by curing the photocationically curable composition, and the viscosity of the photocurable composition, but is substantially free from interacting with the cationically polymerizable monomer (a) and the photoacid generators (b), and hence is a component that is substantially free from affecting the polymerization reaction at the time of photoirradiation, or gelling at the time of long-term storage. Accordingly, even when the filler is further added to the photocationically curable composition according to this embodiment containing no filler, remarkable differences do not occur in curability and storage stability before and after the addition of the filler irrespective of the kind and addition amount of the filler.

7-5. Other Additives

When the photocationically curable composition according to this embodiment is used for a dental curable composition, in particular, a dental filling and restorative material, known other components may be blended thereinto in addition to the above-mentioned components.
Examples of such components include known additives, such as an ultraviolet absorber, a dye, an antistatic agent, a pigment, a fragrance, an organic solvent, and a thickener.

8. Applications of Photocationically Curable Composition according to This Embodiment

Applications of the photocationically curable composition according to this embodiment are not particularly limited, and for example, the photocationically curable composition may be used in various applications, such as an adhesive and a coating material, but in particular, is suitably used in a dental application. In this case, the photocationically curable composition according to this embodiment may be used as a dental curable composition, and in particular, is suitably used as a dental filling and restorative material.
When the photocationically curable composition according to this embodiment is used as a dental curable composition (in particular, a dental filling and restorative material), the photocationically curable composition preferably further contains: an α-diketone compound serving as a photosensitizer; and at least one kind of filler selected from the group consisting of: an inorganic filler; and an organic-inorganic composite filler. In addition, in this case, it is particularly suitable to further satisfy conditions shown in the following <i> to <vi>. More suitable numerical ranges and/or embodiments of the conditions <i> to <iv> and <vi> are similar to those in the above-mentioned cases.

<i> The composition contains a polyfunctional cationically polymerizable monomer having two or more functional groups in the molecule as the cationically polymerizable monomer (a) .
<ii> The content ratio of the polyfunctional cationically polymerizable monomer in the cationically polymerizable monomer (a) is 50 mass% or more.
<iii> The content of the first photoacid generator (b1) with respect to 100 parts by mass of the cationically polymerizable monomer (a) is from 0.01 part by mass to 20 parts by mass.
<iv> The content of the second photoacid generator (b2) with respect to 100 parts by mass of the cationically polymerizable monomer (a) is from 0.01 part by mass to 20 parts by mass.
<v> The content of the α-diketone compound with respect to 100 parts by mass of the cationically polymerizable monomer (a) is from 0.001 part by mass to 10 parts by mass. A more suitable range of the content is from 0.01 part by mass to 5 parts by mass.
<vi> The content of the filler with respect to 100 parts by mass of the cationically polymerizable monomer (a) is from

50 parts by mass to 1,500 parts by mass.

9. Method of producing Photocationically Curable Composition according to This Embodiment

A method of producing the photocationically curable composition according to this embodiment is not particularly limited, and a known curable composition production method may be appropriately adopted. Specifically, it is appropriate that, in a dark place, predetermined amounts of the cationically polymerizable monomer, the photoacid generators, and other blend components to be blended as required, which constitute the curable composition according to this embodiment, be weighed and mixed with each other to give a paste-like mixture. The thus produced photocationically curable composition according to this embodiment is stored under a light-shielding condition until use.

As means for curing the photocationically curable composition according to this embodiment, known polymerization means may be appropriately adopted in accordance with the polymerization initiation mechanisms of the photoacid generator-based photopolymerization initiators used. Specifically, photoirradiation with a light source, such as a carbon arc, a xenon lamp, a metal halide lamp, a tungsten lamp, a fluorescent lamp, sunlight, a helium-cadmium laser, or an argon laser, may be utilized as the polymerization means. In addition, as required, heating treatment using, for example, a heater/polymerizer may also be appropriately utilized in combination with the photoirradiation. An irradiation time for the photoirradiation varies depending on the wavelength and intensity of light to be radiated from the light source, and the shape and material of the cured body, and hence it is appropriate that the irradiation time be determined in advance through a preliminary experiment. However, it is generally preferred that the blending ratios of the various constituent components of the photocationically curable composition be adjusted so that the irradiation time may fall within the range of from about 5 seconds to about 60 seconds.

Examples

The present invention is described below by way of Examples, but the present invention is by no means limited to these Examples.

1. Abbreviations for Compounds

Compounds used in Examples and Comparative Examples and abbreviations therefor are shown below.

(1) Cationically Polymerizable Monomer (a)

·KR-470: a compound represented by the following formula (manufactured by Shin-Etsu Chemical Co., Ltd.)

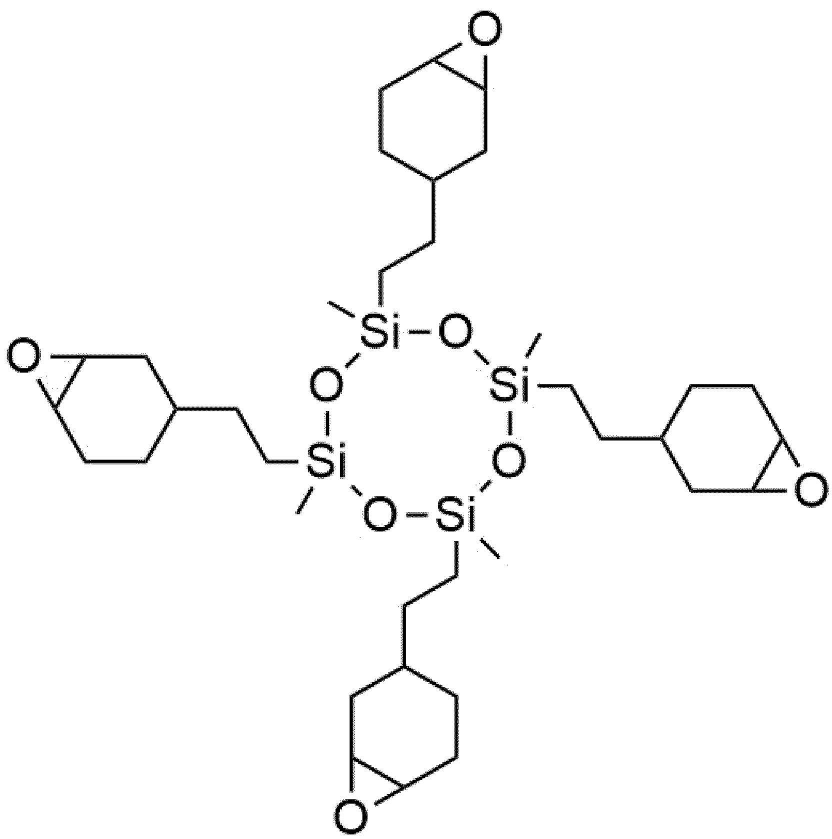

·OXT-121: a compound represented by the following formula (manufactured by Toagosei Co., Ltd.)

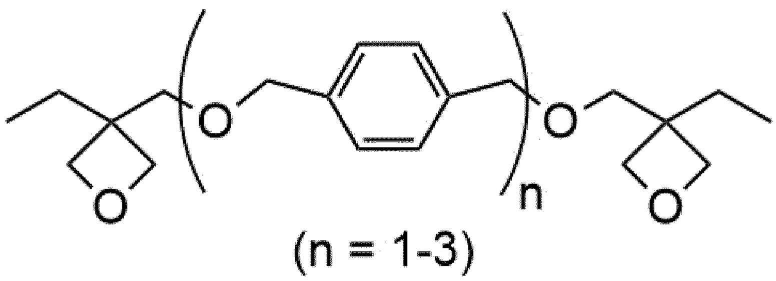

(n = 1-3)

(2) Noncoordinating Anion-type Photoacid Generator (b1)

·DPIB: an iodonium salt using tetrakispentafluorophenylborate as its counteranion, represented by the following formula (manufactured by Tokyo Chemical Industry Co., Ltd.)

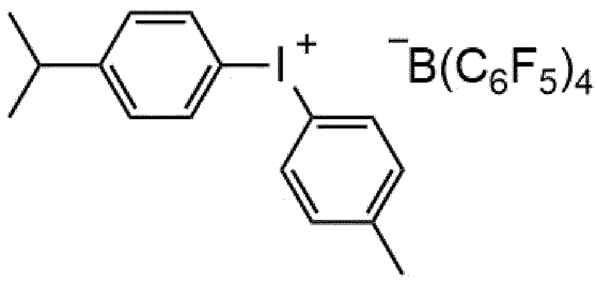

·DPIAl: an iodonium salt using tetrakisnonafluoro-tert-butoxyaluminate as its counteranion, represented by the following formula (manufactured by Tokyo Chemical Industry Co., Ltd.)

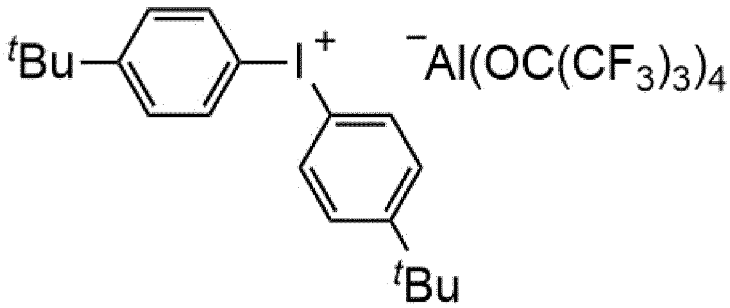

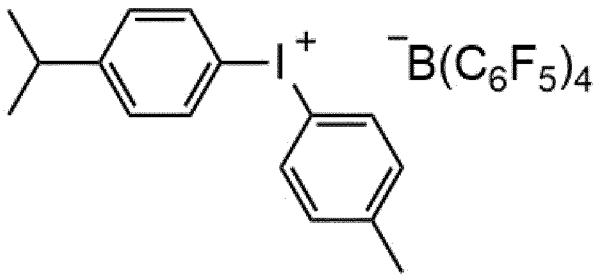

·TPSSb: an iodonium salt using hexafluoroantimonate as its counteranion, represented by the following formula (manufactured by San-Apro Ltd.)

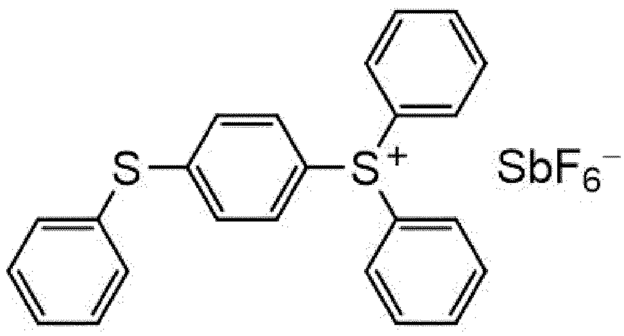

(3) Coordinating Anion-type Photoacid Generator (b2)

·DPII: an iodonium salt using an iodide ion as its counteranion, represented by the following formula (manufactured by Tokyo Chemical Industry Co., Ltd.)

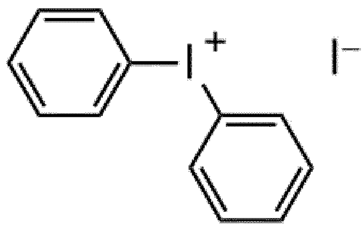

·TPSBr: a sulfonium salt using a bromide ion as its counteranion, represented by the following formula (manufactured by Tokyo Chemical Industry Co., Ltd.)

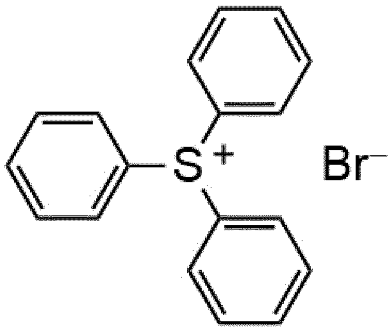

·DPITS: an iodonium salt using p-toluenesulfonate as its counteranion, represented by the following formula (manufactured by Tokyo Chemical Industry Co., Ltd.)

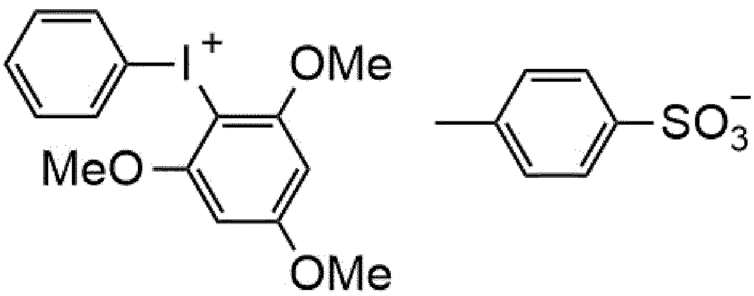

·DPICH: a zwitterionic compound having a benzoate moiety and an iodonium moiety, represented by the following formula (manufactured by Tokyo Chemical Industry Co., Ltd.)

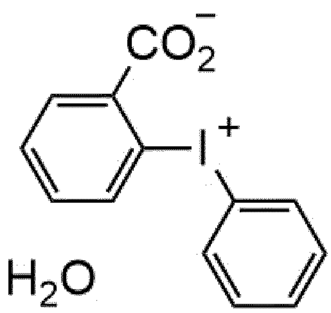

(4) Polymerization Accelerator

·CQ: a compound represented by the following formula (manufactured by Tokyo Chemical Industry Co., Ltd.)

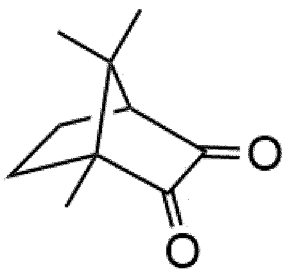

·KC: a compound represented by the following formula (manufactured by Tokyo Chemical Industry Co., Ltd.)

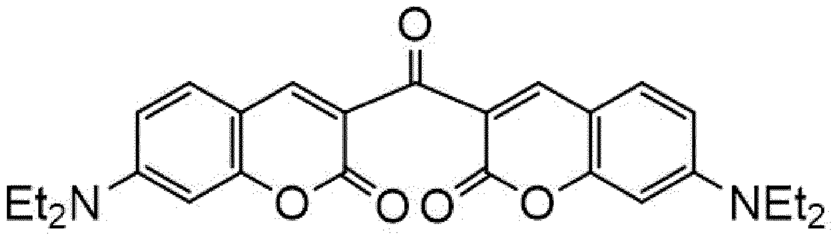

·DMBE: a compound represented by the following formula (manufactured by Tokyo Chemical Industry Co., Ltd.)

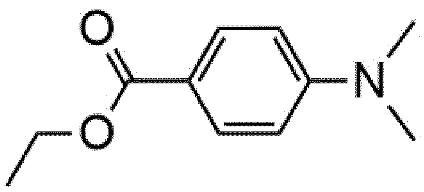

·EDMAn: a compound represented by the following formula (manufactured by Sigma-Aldrich Co. LLC)

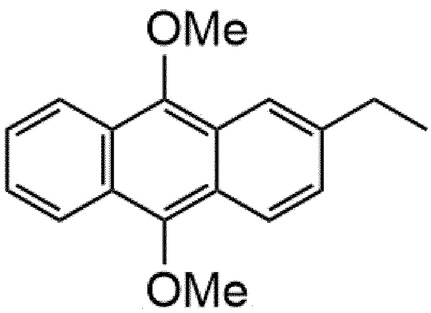

(5) Phenolic Antioxidant

·BHT: a compound represented by the following formula (manufactured by Tokyo Chemical Industry Co., Ltd.)

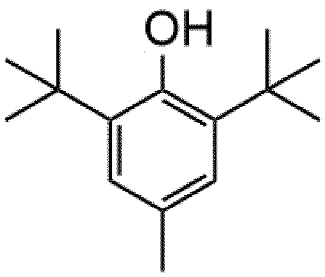

·HQME: a compound represented by the following formula (manufactured by Fujifilm Wako Pure Chemical Corporation)

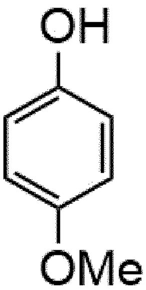

(6) Hydrogen Ion Scavenger

·TEATS: a compound represented by the following formula (manufactured by Tokyo Chemical Industry Co., Ltd.)

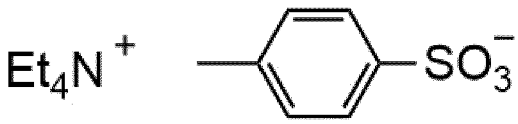

·TN770: a compound represented by the following formula (manufactured by BASF Japan Ltd.)

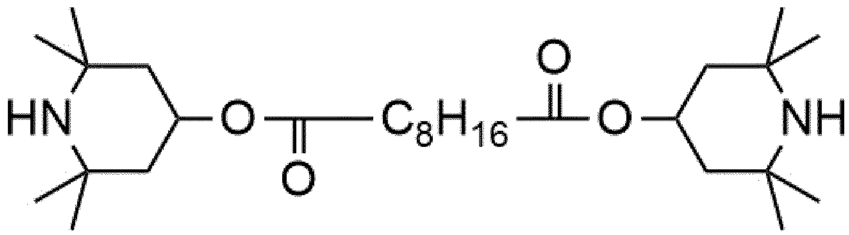

2. Examples and Comparative Examples

Example 1

1.4 Parts by mass of DPIPB (1.4 mmol/kilogram) and 0.32 part by mass of DPICH (1.0 mmol/kilogram) serving as photoacid generators, 0.3 part by mass of CQ and 0.5 part by mass of DMBE serving as polymerization accelerators, and 0.2 part by mass of BHT serving as a phenolic antioxidant were added to 100 parts by mass of cationically polymerizable monomers including 50 parts by mass of KR-470 and 50 parts by mass of OXT-121, and the mixture was stirred under red light for 6 hours to prepare a photocationically curable composition.

The resultant photocationically curable composition was evaluated for its curing time and gelling time by methods described below. The results are shown in Table 1.

(1) Curability Evaluation (Curing Time)

The prepared photocationically curable composition was filled into a mold made of polyacetal having a hole with a diameter of 7 mm and a depth of 1.0 mm. Then, through use of a visible light photoirradiator for dental use (TOKUSO POWER LITE, manufactured by Tokuyama Corporation), the filling matter filled into the hole was subjected to photoirradiation under room temperature for a given time period (light intensity on irradiation surface: 400 $mW/cm^2$). Immediately after the photoirradiation, the state of the filling matter was checked to check whether the entire photocationically curable composition was cured. When the entire photocationically curable composition was cured, the cured body was taken out of the mold, and its strength was checked by pinching both ends thereof with fingers to lightly apply a force. In the case where there was an uncured portion at the time of the checking after the photoirradiation, or in the case where the cured body was easily bent or broken when a force was lightly applied with fingers, the curing was regarded as insufficient, and in the case where the cured body was not bent or broken even when a force was applied with fingers, the curing was regarded as satisfactory. First, the above-mentioned test was performed with the irradiation

time being 1 second, and if the curing was insufficient, filling work was freshly performed, and then the test was repeatedly performed with the irradiation time being appropriately extended, until the curing became satisfactory. Then, when the irradiation time was increased in the order of 1 second, 2 seconds, 3 seconds, 5 seconds, 10 seconds, 20 seconds, 30 seconds, 40 seconds, 50 seconds, and 60 seconds, the irradiation time at which the curing first became satisfactory was defined as the curing time. Here, a case in which the curing was insufficient when the irradiation time was 60 seconds was judged as unevaluable.

For reference, when the photocationically curable composition is used as a dental curable composition, the curing time is generally preferably 40 seconds or less, more preferably 20 seconds or less. In particular, when the photocationically curable composition is used as a dental filling and restorative material (so-called composite resin), photoirradiation needs to be performed with a photoirradiator inserted into the mouth of a patient. For this reason, the ability to control the curing time to 40 seconds or less is extremely beneficial in that burdens on both the patient and a practitioner can be reduced.

(2) Color of Cured Body

The color of the cured body obtained at the irradiation time at which the curing first became satisfactory in the foregoing section "(1) Curability Evaluation (Curing Time)" was visually determined.

(3) Storage Stability Evaluation (Gelling Time)

0.3 g of the prepared photocationically curable composition was placed in a 6 mL brown sample vial and stored in a thermostatic apparatus at 60°C under a light-shielding condition. The photocationically curable composition was taken out of the thermostatic apparatus at 1 day, 2 days, 3 days, 4 days, 5 days, 7 days, 10 days, 14 days, and every 7 days thereafter, and after standing to cool under a dark place to room temperature, the nature of the photocationically curable composition was observed. In this case, the number of days at the time point when the photocationically curable composition became a gel that did not flow at all even by tilting the sample vial was defined as the gelling time. When gelling did not occur even after 60 days, observation was terminated (shown as ">60 days" in the tables). When no change was found in nature of the photocationically curable composition at the time point of having been stored at 60°C for 14 days in this test, the photocationically curable composition is expected to be storable for roughly 1 year or more when stored at room temperature.

Examples 2 to 8 and Comparative Examples 1 to 7

Photocationically curable compositions were prepared in the same manner as in Example 1 except that photoacid generators and a hydrogen ion scavenger to be blended were changed as shown in Table 1, and were each evaluated for its curability and gelling time in the same manner as in Example 1. The results are shown in Table 1.

Table 1

| | b: Photoacid generators | | | | | | | Hydrogen ion scavenger | | Curing time | Gelling time |
|---|---|---|---|---|---|---|---|---|---|---|---|
| | b1: Noncoordinating anion type | | | b2: Coordinating anion type | | | | | | | |
| | DPIB | DPIAl | TPSSb | DPII | TPSBr | DPITS | DPICH | TEATS | TN770 | | |
| Example 1 | 1.4 (1.4) | - | - | - | - | - | 0.32 (1.0) | - | - | 10 seconds | 35 days |
| Example 2 | 1.4 (1.4) | - | - | - | - | 0.54 (1.0) | - | - | - | 5 seconds | 42 days |
| Example 3 | 1.4 (1.4) | - | - | - | 0.34 (1.0) | - | - | - | - | 10 seconds | 28 days |
| Example 4 | 1.4 (1.4) | - | - | 0.41 (1.0) | - | - | - | - | - | 5 seconds | 21 days |
| Example 5 | - | 1.9 (1.4) | - | - | - | - | 0.32 (1.0) | - | - | 10 seconds | 42 days |
| Example 6 | - | 1.9 (1.4) | - | 0.41 (1.0) | - | - | - | - | - | 10 seconds | 28 days |
| Example 7 | - | - | 0.8 (1.4) | - | - | - | 0.32 (1.0) | - | - | 20 seconds | 35 days |
| Example 8 | - | - | 0.8 (1.4) | - | - | 0.54 (1.0) | - | - | - | 10 seconds | 35 days |
| Comparative Example 1 | 1.4 (1.4) | - | - | - | - | - | - | - | - | 2 seconds | 7 days |
| Comparative Example 2 | 1.4 (1.4) | 1.3 (1.0) | 1.3 (1.0) | - | - | - | - | - | - | 2 seconds | 4 days |
| Comparative Example 3 | - | - | - | 0.57 (1.4) | | - | - | - | - | Unevaluable | >60 days |
| Comparative Example 4 | - | - | - | 0.57 (1.4) | - | - | 0.32 (1.0) | - | - | Unevaluable | >60 days |
| Comparative Example 5 | 1.4 (1.4) | - | - | - | - | - | - | 0.29 (1.0) | - | 50 seconds | 42 days |
| Comparative Example 6 | 1.4 (1.4) | - | - | - | - | - | - | - | 0.24 (1.0) | 50 seconds | 21 days |
| Comparative Example 7 | 1.4 (1.4) | - | - | - | - | - | 0.16 (0.5) | - | 0.12 (0.5) | 40 seconds | 21 days |

*A numerical value in the table represents the mass% of each component with respect to 100 of the cationically polymerizable monomers
*A numerical value in parentheses in the table represents the amount of substance/mmol of each component contained in 1 kg of the composition
·Cationic monomers: KR-470 (50 parts by mass) and OXT-121 (50 parts by mass)
·Polymerization accelerators: CQ (0.3 part by mass) and DMBE (0.5 part by mass)
·Phenolic antioxidant: BHT (0.2 part by mass)

As shown in Examples 1 to 8 of Table 1 above, when the noncoordinating anion-type photoacid generator (b1) and the coordinating anion-type photoacid generator (b2) were blended in combination, curing occurred within 20 seconds after photoirradiation, and besides, the number of days of storage until gelling occurred under storage at 60°C was relatively large. In contrast, when only the noncoordinating anion-type photoacid generator (b1) was blended (Comparative Examples 1 and 2), although the curing rate was high, gelling occurred within 7 days. In addition, when only the coordinating anion-type photoacid generator (b2) was blended (Comparative Examples 3 and 4), curing was insufficient even after 60 seconds of photoirradiation. In addition, when the hydrogen ion scavenger was blended (Comparative Examples 5 to 7), although a suppressive effect on gelling was found, the curing rate was significantly reduced.

Examples 9 to 20 and Comparative Examples 8 to 12

Photocationically curable compositions were prepared in the same manner as in Example 1 except that blend components and blending amounts were changed as shown in Table 2, and were each evaluated for its curing time and gelling time in the same manner as in Example 1. The results are shown in Table 2.

Table 2

| | b: Photoacid generators | | | Polymerization accelerator | | | | Phenolic antioxidant | | Curing time | Color of cured body | Gelling time |
|---|---|---|---|---|---|---|---|---|---|---|---|---|
| | b1: Noncoordinating anion type | b2: Coordinating anion type | | | | | | | | | | |
| | DPIB | DPITS | DPICH | CQ | KC | DMBE | EDMAn | BHT | HQME | | | |
| Example 9 | 3.0 (3.0) | - | 0.05 (0.15) | 0.3 (1.8) | - | 0.5 (2.6) | - | 0.2 (0.9) | - | 2 seconds | Pale yellow | 14 days |
| Example 10 | 3.0 (3.0) | - | 0.10 (0.3) | 0.3 (1.8) | - | 0.5 (2.6) | - | 0.2 (0.9) | - | 2 seconds | Pale yellow | 21 days |
| Example 11 | 3.0 (3.0) | - | 0.32 (1.0) | 0.3 (1.8) | - | 0.5 (2.6) | - | 0.2 (0.9) | - | 3 seconds | Pale yellow | 28 days |
| Example 12 | 3.0 (3.0) | - | 0.32 (1.0) | 0.3 (1.8) | - | - | 0.5 (1.5) | 0.2 (0.9) | - | 2 seconds | Pale yellow | 28 days |
| Example 13 | 3.0 (3.0) | - | 0.32 (1.0) | - | 0.3 (0.7) | - | 0.5 (1.5) | 0.2 (0.9) | - | 3 seconds | Orange | 28 days |
| Example 14 | 3.0 (3.0) | - | 0.32 (1.0) | - | - | - | 0.5 (1.5) | 0.2 (0.9) | - | 5 seconds | Pale yellow | 28 days |
| Example 15 | 1.4 (1.4) | 0.38 (0.7) | - | 0.3 (1.8) | - | 0.5 (2.6) | - | 0.2 (0.9) | - | 3 seconds | Pale yellow | 35 days |
| Example 16 | 1.4 (1.4) | 0.65 (1.2) | - | 0.3 (1.8) | - | 0.5 (2.6) | - | 0.2 (0.9) | - | 10 seconds | Pale yellow | 42 days |
| Example 17 | 1.4 (1.4) | 0.54 (1.0) | - | 0.3 (1.8) | - | 0.5 (2.6) | - | - | 0.1 (0.8) | 5 seconds | Pale yellow | 35 days |
| Example 18 | 1.4 (1.4) | 0.54 (1.0) | - | 0.3 (1.8) | - | 0.5 (2.6) | - | - | - | 3 seconds | Pale yellow | 14 days |
| Example 19 | 1.0 (1.0) | - | 0.03 (0.10) | 0.1 (1.8) | - | 0.2 (2.6) | - | 0.05 (0.2) | - | 5 seconds | Pale yellow | 14 days |
| Example 20 | 4.0 (4.0) | 1.08 (2.0) | - | 0.5 (1.8) | - | 1.0 (2.6) | - | 0.5 (2.3) | - | 10 seconds | Pale yellow | 28 days |
| Comparative Example 8 | 3.0 (3.0) | - | - | 0.3 (1.8) | - | 0.5 (2.6) | - | 0.2 (0.9) | - | 1 second | Pale yellow | 4 days |

(continued)

| | b: Photoacid generators | | | Polymerization accelerator | | | | Phenolic antioxidant | | Curing time | Color of cured body | Gelling time |
|---|---|---|---|---|---|---|---|---|---|---|---|---|
| | b1: Noncoordinating anion type | b2: Coordinating anion type | | | | | | | | | | |
| | DPIB | DPITS | DPICH | CQ | KC | DMBE | EDMAn | BHT | HQME | | | |
| Comparative Example 9 | - | 1.0 (3.0) | - | 0.3 (1.8) | - | 0.5 (2.6) | - | 0.2 (0.9) | - | Unevaluable | Pale yellow | >60 days |
| Comparative Example 10 | 1.4 (1.4) | - | - | - | - | - | 0.5 (1.5) | 0.2 (0.9) | - | 3 seconds | Pale yellow | 4 days |
| Comparative Example 11 | 1.4 (1.4) | - | - | 0.3 (1.8) | - | 0.5 (2.6) | - | - | 0.1 (0.8) | 2 seconds | Pale yellow | 7 days |
| Comparative Example 12 | 1.4 (1.4) | - | - | 0.3 (1.8) | - | 0.5 (2.6) | - | - | - | 1 second | Pale yellow | 2 days |

*A numerical value in the table represents the mass% of each component with respect to 100 of the cationically polymerizable monomers
*A numerical value in parentheses in the table represents the amount of substance/mmol of each component contained in 1 kg of the composition
·Cationic monomers: KR-470 (50 parts by mass) and OXT-121 (50 parts by mass)

As shown in Examples 9 to 20 of Table 2 above, when the noncoordinating anion-type photoacid generator (b1) and the coordinating anion-type photoacid generator (b2) were blended in combination, curing occurred within 10 seconds after photoirradiation, and besides, the number of days of storage until gelling occurred under storage at 60°C was relatively large. In addition, in a comparison among Examples 12 to 14, the curing rate was more satisfactory when CQ or KC serving as a photosensitizer was added (Examples 12 and 13) than when no photosensitizer was added (Example 14). When CQ was added as a photosensitizer (Example 12), the cured body showed a pale yellow color, but when KC was added (Example 13), the cured body showed an orange color. Meanwhile, when only the noncoordinating anion-type photoacid generator (b1) was blended (Comparative Examples 8 and 10 to 12), although the curing rate was high, gelling occurred within 7 days. In addition, when only the coordinating anion-type photoacid generator (b2) was blended (Comparative Example 9), curing was insufficient even after 60 seconds of photoirradiation.

The disclosures of Non Patent Literature 1 and Non Patent Literature 2 are incorporated herein by reference in their entirety.

## Claims

1. A photocationically curable composition, comprising:

    a cationically polymerizable monomer (a); and
    photoacid generators (b),
    the photocationically curable composition being substantially free of a hydrogen ion scavenger,
    wherein the photoacid generators (b) include:

    at least one kind of first photoacid generator (b1) selected from the group consisting of:

    a photoacid generator (b1-I) formed of an ion pair of an anion and a cation, the anion being a noncoordinating anion; and
    a photoacid generator (b1-II) formed of a zwitterionic compound having an anionic moiety and a cationic moiety in a molecule, the anionic moiety being a noncoordinating anionic moiety; and

    at least one kind of second photoacid generator (b2) selected from the group consisting of:

    a photoacid generator (b2-I) formed of an ion pair of an anion and a cation, the anion being an anion other than a noncoordinating anion; and
    a photoacid generator (b2-II) formed of a zwitterionic compound having an anionic moiety and a cationic moiety in a molecule, the anionic moiety being an anionic moiety other than a noncoordinating anionic moiety.

2. The photocationically curable composition according to claim 1, wherein the noncoordinating anion in the first photoacid generator (b1) is at least one kind of anion selected from the group consisting of: (a) perchlorate; (b) trifluoromethanesulfonate; (c) $BX_4^-$; (d) $AlX_4^-$; (e) $PX_6^-$; (f) $AsX_6^-$; and (g) $SbX_6^-$, where X in each of the chemical formulae shown in (c) to (g) represents a fluoro group, an alkyl group substituted with one or more fluorine atoms, an alkoxy group substituted with one or more fluorine atoms, or an aryl group substituted with one or more fluorine atoms, and a plurality of Xs included in each of the chemical formulae may be identical to or different from each other.

3. The photocationically curable composition according to claim 1 or 2, wherein the cationically polymerizable monomer (a) includes at least one kind of compound selected from the group consisting of: an epoxy compound; and an oxetane compound.

4. The photocationically curable composition according to any one of claims 1 to 3, wherein a blending amount of the second photoacid generator (b2) with respect to 1 mol of a blending amount of the first photoacid generator (b1) included in the photoacid generators (b) is from 0.1 mol to 0.75 mol.

5. The photocationically curable composition according to any one of claims 1 to 4, wherein the second photoacid generator (b2) is at least one kind of salt selected from the group consisting of: an iodonium salt; and a sulfonium salt.

6. The photocationically curable composition according to any one of claims 1 to 5, further comprising at least one kind of component selected from the group consisting of: a photosensitizer; an electron-donating compound; a

phenolic antioxidant; and a filler.

**7.** A dental curable composition, comprising:

a cationically polymerizable monomer (a); and
photoacid generators (b),
the dental curable composition being substantially free of a hydrogen ion scavenger,
wherein the photoacid generators (b) include:

at least one kind of first photoacid generator (b1) selected from the group consisting of:

a photoacid generator (b1-I) formed of an ion pair of an anion and a cation, the anion being a noncoordinating anion; and
a photoacid generator (b1-II) formed of a zwitterionic compound having an anionic moiety and a cationic moiety in a molecule, the anionic moiety being a noncoordinating anionic moiety; and

at least one kind of second photoacid generator (b2) selected from the group consisting of:

a photoacid generator (b2-I) formed of an ion pair of an anion and a cation, the anion being an anion other than a noncoordinating anion; and
a photoacid generator (b2-II) formed of a zwitterionic compound having an anionic moiety and a cationic moiety in a molecule, the anionic moiety being an anionic moiety other than a noncoordinating anionic moiety.

**8.** The dental curable composition according to claim 7, further comprising:

an $\alpha$-diketone compound (c) serving as a photosensitizer; and
at least one kind of filler (d) selected from the group consisting of: an inorganic filler; and an organic-inorganic composite filler.

**9.** The dental curable composition according to claim 8,

wherein the dental curable composition comprises a polyfunctional cationically polymerizable monomer having two or more functional groups in a molecule as the cationically polymerizable monomer (a),
wherein a content ratio of the polyfunctional cationically polymerizable monomer in the cationically polymerizable monomer (a) is 50 mass% or more,
wherein a content of the first photoacid generator (b1) with respect to 100 parts by mass of the cationically polymerizable monomer (a) is from 0.01 part by mass to 20 parts by mass,
wherein a content of the second photoacid generator (b2) with respect to 100 parts by mass of the cationically polymerizable monomer (a) is from 0.01 part by mass to 20 parts by mass,
wherein a content of the $\alpha$-diketone compound (c) with respect to 100 parts by mass of the cationically polymerizable monomer (a) is from 0.001 part by mass to 10 parts by mass, and
wherein a content of the filler (d) with respect to 100 parts by mass of the cationically polymerizable monomer (a) is from 50 parts by mass to 1,500 parts by mass.

## INTERNATIONAL SEARCH REPORT

International application No. PCT/JP2021/027481

A. CLASSIFICATION OF SUBJECT MATTER
Int.Cl. C08G59/68(2006.01)i, A61K6/61(2020.01)i, A61K6/891(2020.01)i
FI: C08G59/68, A61K6/61, A61K6/891

According to International Patent Classification (IPC) or to both national classification and IPC

B. FIELDS SEARCHED

Minimum documentation searched (classification system followed by classification symbols)
Int.Cl. C08G59/68, A61K6/61, A61K6/891

Documentation searched other than minimum documentation to the extent that such documents are included in the fields searched

| | |
|---|---|
| Published examined utility model applications of Japan | 1922-1996 |
| Published unexamined utility model applications of Japan | 1971-2021 |
| Registered utility model specifications of Japan | 1996-2021 |
| Published registered utility model applications of Japan | 1994-2021 |

Electronic data base consulted during the international search (name of data base and, where practicable, search terms used)
CAplus/REGISTRY(STN)

C. DOCUMENTS CONSIDERED TO BE RELEVANT

| Category* | Citation of document, with indication, where appropriate, of the relevant passages | Relevant to claim No. |
|---|---|---|
| X<br>A | JP 5-5006 A (NIPPON KAYAKU CO., LTD.) 14 January 1993 (1993-01-14), claims, paragraphs [0001], [0007], [0010], [0012], examples, tables 1-15 | 1-6<br>7-9 |
| X<br>A | JP 2016-64641 A (CANON INC.) 28 April 2016 (2016-04-28), claims, paragraphs [0044]-[0046], table 4 | 1-3, 5, 6<br>4, 7-9 |
| A | JP 2001-520759 A (MINNESOTA MINING AND MANUFACTURING COMPANY) 30 October 2001 (2001-10-30), entire text | 1-9 |
| E, X | JP 2021-116356 A (TOKUYAMA DENTAL CORPORATION) 10 August 2021 (2021-08-10), claims, paragraphs [0052]-[0071], [0074]-[0111], tables 1-3 | 1-9 |

☐ Further documents are listed in the continuation of Box C.  ☒ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 13 September 2021 | 21 September 2021 |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

**INTERNATIONAL SEARCH REPORT**
Information on patent family members

International application No.
PCT/JP2021/027481

| | | |
|---|---|---|
| JP 5-5006 A | 14 January 1993 | US 5359017 A<br>claims, column 1,<br>lines 10-20, column 2,<br>lines 33-54, column 3,<br>lines 9-23, column 3,<br>line 62 to column 4, line 2,<br>examples, tables 1-15<br>EP 511405 A1 |
| JP 2016-64641 A | 28 April 2016 | US 2016/0091789 A1<br>claims, paragraph [0047],<br>table 4<br>EP 3001248 A1<br>CN 105467757 A |
| JP 2001-520759 A | 30 October 2001 | US 5998495 A<br>entire text<br>CN 1252137 A<br>KR 10-2001-0006281 A |
| JP 2021-116356 A | 10 August 2021 | (Family: none) |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2004196775 A
- JP 10508067 A
- JP 2005523348 A
- JP 2002069269 A
- JP 2006249040 A
- JP 2007131841 A


### Non-patent literature cited in the description


- **KROSSING, I. ; RAABE, I.** Noncoordinating Anions-Fact or Fiction? A Survey of Likely Candidates. *Angew. Chem. Int. Ed.,* 2004, vol. 43, 2066-2090
- **RIDDLESTONE, IAN M. ; KRAFT, A. ; SCHAEFER, J. ; KROSSING, I.** Taming the Cationic Beast: Novel Developments in the Synthesis and Application of Weakly Coordinating Anions. *Angew. Chem. Int. Ed.,* 2018, vol. 57, 13982-14024
- *J Polym Sci Part A: Polym Chem,* 2003, vol. 41, 2570